# EUROPEAN PATENT SPECIFICATION

(11) **EP 0 726 805 B1**
(45) Date of publication and mention of the grant of the patent: **11.06.1997**
(21) Application number: 93912047.3
(22) Date of filing: 27.04.1993
(51) Int. Cl.: B01F 3/12, B01F 13/06, A61F 2/46

(54) **DEVICE FOR PREPARING BONE CEMENT**
VORRICHTUNG ZUR HERSTELLUNG VON KNOCHENZEMENT
DISPOSITIF DE PREPARATION DE CIMENT POUR OS

(30) Priority: 29.04.1992 SE 9201353
(43) Date of publication of application: 21.08.1996
(73) Proprietor: CEMVAC SYSTEM AKTIEBOLAG, 589 41 Linköping (SE)
(72) Inventor: JONSSON, Sören, S-582 70 Linköping (SE); SMEDS, Staffan, S-585 99 Linköping (SE); THORLING, Jan, S-791 91 Falun (SE); NILSSON, Thomas, S-311 45 Falkenberg (SE); BERGMAN, Torvald, S-194 37 Upplands Väsby (SE)
(74) Representative: Willquist, Bo
(86) International application number: SE9300366
(87) International publication number: WO9322041

(56) References cited:
- SE-B- 462 315
- Patent Abstracts of Japan, vol. 10, no. 292, C-376, abstract of JP,A, 61-111130 (OKI ELECTRIC IND CO LTD), 29 May 1986 (29.05.86)

## Description

The present invention relates to an arrangement for preparation of bone cement, which bone cement comprises at least two constituent components, said arrangement including a cylindrical chamber, which has introduction means for introduction of the constituents, which are to be mixed in the chamber, and closure means for closing the chamber after introduction of said constituents, whereby one end of the chamber is closed by a slidable piston for ejection of the mixture, and the other end is closed by a lid, which includes a connection for a vacuum-suction device, as well as a central aperture for a detachable agitator rod, which is attached to an agitator device, axially movable within the chamber.

The invention also relates to a method for the preparation of bone cement, composed of at least two constituent components, by mixing of the constituents whereby the constituents are fed to a cylindrical chamber, which is closed after the introduction of said constituents, and whereby one end of the chamber is closed by means of a slidable piston for ejection of the mixture, and the other end is closed by a lid, which includes a connection for a vacuum-suction device as well as a central aperture for a detachable agitator rod which is dismountably attached to an agitator device, axially movable within the chamber.

When preparing bone cement, which for instance is used for attachment of hip-joint protheses ,toxic or in other ways environmentally hazardous gases occur, which causes problems. Bone cement is normally prepared through mixing of powdered polymetylmethacrylate and liquid methylmethacrylate monomer. It is important that the mixture should be as homogeneous as possible, since lack of homogeneity and gas inclusions have the effect of reducing the strength of the bone cement. Gases which occur during the preparation are odourous and noxious.

SE, B, 450545 discloses a method and a device for the preparation of bone cement. The substances to be mixed are placed in an open vessel, which thereafter is closed, and vacuum is applied for withdrawing surplus gases from the mixing vessel. However, this known device does not solve the problem of gasdischarge during the open time of the vessel.

WO, A, 9013264 discloses other arrangements for mixing, which also employs vacuum for surplus gas withdrawal. One of the embodiments discloses a device in which the introduction of the bone cement constituents is achieved through the hollow agitator rod. Thus, introduction takes place through a relatively long and narrow channel, which renders the introduction more difficult.

The object of the present invention is to make available an arrangement according to the introductory parts of the description, which facilitates introduction, with minimal risk of gas discharge.

For this purpose, the invention is characterized in that said introduction means include a single inlet separate from said central aperture with an introduction funnel which, at least during an introductory course, is hermetically connected to said single inlet, and that the vacuum-suction device is intended to produce a flow of air from the surroundings through the funnel, the inlet and the chamber and forward towards the connection.

A method according to the invention is characterized in that the introduction of said constituents takes place by means of a single inlet, which single inlet is separate from the central aperture, with an introduction funnel which is hermetically connected to said single inlet, at least during an introduction process, and that during vacuum-suction a flow of air is produced from the surroundings through the funnel and the inlet, into the chamber and towards the connection.

Preferred embodiments of the invention are apparent from the accompanying dependent claims.

One embodiment of the invention will now be further described with reference to the accompanying drawings in which, Figure 1 is a side view of an embodiment of a mixer according to the invention, prepared for introduction of the constituent components, Figure 2 illustrates a plug for closing the inlet of the chamber, Figure 3 illustrates an ejection tube and Figure 4 illustrates the mixing agitator device viewed from below. Figure 5 is a longitudinal section of an alternative embodiment of the mixer according to the invention, with an inlet in the shape of a channel, placed on the outside of the cylindrical chamber and emerging into the chamber from the cylindrical wall.

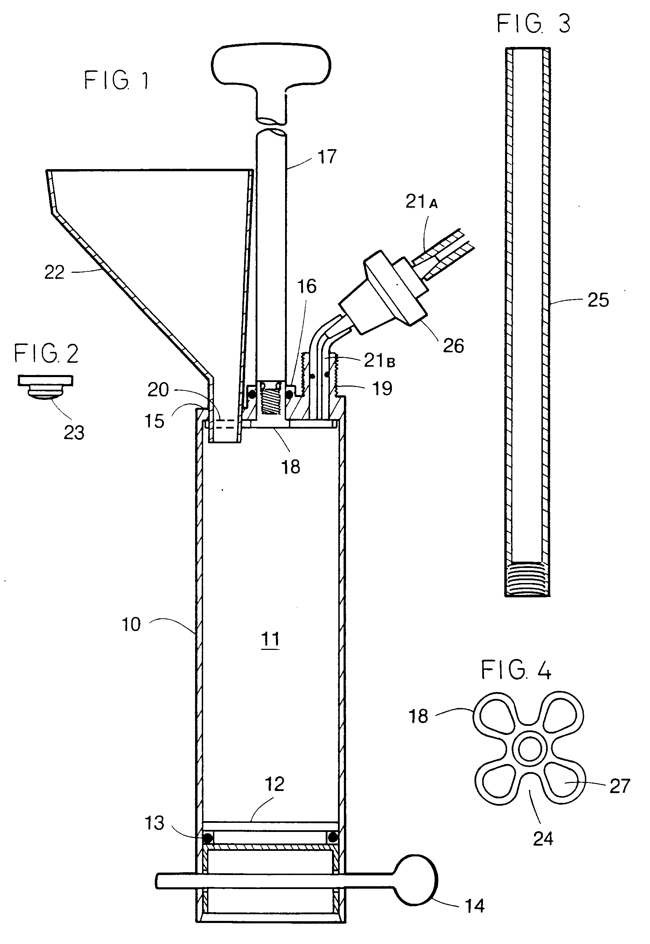

In the drawings, the number 10 denotes a mixing cylinder with an inner chamber 11, which has one end closed by a piston 12, equipped with a gasket ring 13 and in a position of application, is slidable into the chamber 11. In Figure 1 the piston 12 is locked by means of a cotter pin 14, which extends through the cylinder 10 as well as the piston 12.

The other end of the chamber 11 is closed by means of a lid 15, preferably integrated with the cylinder 10, and presents a central aperture with a gasket ring against an agitator rod 17. This rod is demountably attached to an agitator rod, movable within the chamber 11.

Furthermore the lid 15 is provided with a fixed connection nipple 19. In addition there is an inlet 20 for the constituent components to be mixed in the chamber 11.

There is a demountable, tightly connected choke 21B in the connection nipple 19, which is joined to a filter 26. The choke 21B is axially movable in and out of the chamber 11.

The mixing device is in Figure 1 displayed in a position for introduction of the constituent components; which means that the nipple 19, the choke 21B and the filter 26 are joined, by means of a tube 21A, to a non-displayed vacuum-suction device, which by the filter 26 is prevented from being contaminated by the substances added to the chamber. The inlet 20 has been provided with an hermetically connected funnel. The vacuum-suction device is arranged to generate a negative pressure in the chamber 11, which propagates to the funnel 22.

The negative pressure generates a flow of air from the surroundings through the funnel 22, and sucks down the constituent components to be mixed in the chamber 11, and at the same time vapors that are produced from these components. Noxious gases are in this way prevented from spreading to the surroundings. The non-displayed vacuum-suction device preferably contains active carbon, to effectively purify said gases, and may also be supplemented with a so called sterile filter.

Subsequently to the introduction of substances, the funnel 22 is removed and an airtight plug 23, disclosed in Figure 2, is inserted into the inlet 20. The mixing may now be accomplished, and in the meantime, suction is continued of the formed gases with an increased negative pressure via the choke 21B, the filter 26 and the tube 21A, whereby a continuous suction flow is established. The mixing procedure itself is accomplished by moving the agitator device 18 up and down in the chamber by means of the agitator rod during simultaneous twisting.

After approximately twenty pumping movements according to above, the agitator rod 17 is pulled towards the lid 15, the agitator device 18 is twisted until one of the notches 24 or one of the cavities 27, is in front of the choke 21B, which now may be pushed down or screwed down to lock the agitator device 18 against turning. The agitator rod 17 may now be unscrewed from the agitator device 18, which closes the aperture 16.

The tube 21A, the filter 26 and the choke 21B can be demounted from the nippel 19, after which the ejection nozzle, illustrated in Figure 3, is screwed onto the nippel 19. After removal of the cotter pin 14, the mixer is placed in a known syringe mechanism for ejection of the mixed content in chamber 11, by means of the ejection nozzle 25.

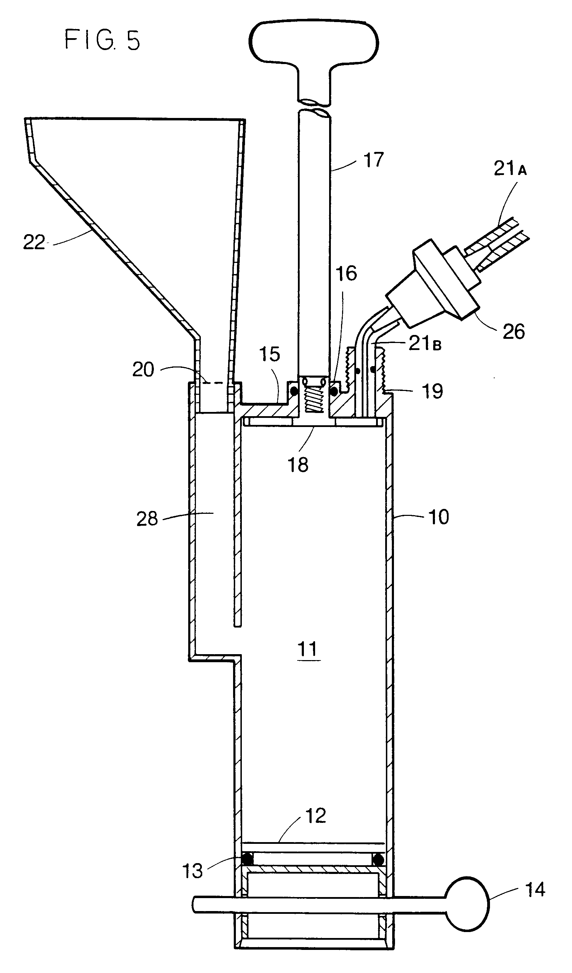

For the embodiment of a mixing device according to the invention illustrated in Figure 5, all that was indicated for the embodiment according to Figure 1 is valid, except that the inlet 20 for the constituents intended for mixing in the chamber 11, is placed on the cylindrical wall of the chamber.

The inlet 20 communicates with the funnel 22 via a channel 28 placed on the outside of the chamber wall. An advantage of this embodiment is that a certain premixture of the constituent components of bone cement is achieved, due to the fact that the first substance, by its level in the chamber, covers the inlet 20. When the second substance is introduced, it will be forced to pass through the first substance.

For instance, it is not necessary for the lid 15 to be manufactured as an integrated part of the mixing cylinder, it may for production reasons be manufactured as a detachable part which can be screwed onto the mixer cylinder.

## Claims

1. Arrangement for the preparation of bone cement, which bone cement comprises at least two constituent components, said arrangement including a cylindrical chamber (11), which has introduction means for the introduction of the constituents, which are to be mixed in the chamber, and closure means (23) for closing the chamber (11) after the introduction of said constituents, whereby one end of the chamber is closed by means of a slidable piston (12) for ejection of the mixture, and the other end is closed by a lid (15), which includes a connection (19) for a vacuum-suction device (21), as well as a central aperture (16) for a detachable agitator rod (17), which is attached to an agitator device (18), axially movable within the chamber, **characterized** in that said introduction means include a single inlet (20) separate from said central aperture (16) with an introduction funnel (22) which, at least during an introduction process, is hermetically connected to said inlet (20), and that the vacuum-suction device is intended to produce a flow of air from the surroundings through the funnel (22), the inlet (20) and the chamber (11) and forward towards the connection (19).

2. Arrangement according to claim 1, **characterized** in that the vacuum-suction device includes a purification filter (26) containing active carbon and a sterile filter.

3. Arrangement according to any of the preceeding claims, **characterized** in that the connection (19) following dismounting of the choke (21B), forms a firmament for an ejection nozzle.

4. Arrangement according to any of the preceeding claims, **characterized** in that the inlet (20) emerges into the lid (15).

5. Arrangement according to any of the claims 1-3, **characterized** in that the inlet (20) emerges into the cylindrical wall of the chamber (11) at such a distance from the piston (12) that the inlet is covered by the first constituent component after the introduction of said constituent.

6. Arrangement according to any of the preceeding claims, **characterized** in that the choke (21B) is slidable into the mixing chamber (11).

7. Method for the preparation of bone cement, composed of at least two constituent components, by mixing of the constituents, whereby the constituents are fed to a cylindrical chamber (11), which is closed after the introduction of said constituents, and whereby one end of the chamber (11) is closed by means of a slidable piston (12) for ejection of the mixture, and the other end is closed by a lid (15), which includes a connection (19) for a vacuum-suction device (21), as well as a central aperture (16) for a detachable agitator rod (17), which is dismountably attached to an agitator device (18), axially movable within the chamber, **characterized** in that the introduction of said constituents takes place by means of a single inlet (20), which single inlet (20) is separate from the central aperture (16), with an introduction funnel (22) which is hermetically connected to said single inlet (20), at least during an introduction process, and that during vacuum-suction a flow of air is produced from the surroundings through the funnel (22), the inlet (20) and the chamber (11) and towards the connection (19).

8. Method according to claim 7, **characterized** in that the introduction of said constituents takes place by means of an inlet (20) in the cylindrical wall of the chamber (11), after introduction of the first constituent said inlet is below the level of the first constituent in the chamber (11).

## Patentansprüche

1. Anordnung zum Herstellen von Knochenzement, der wenigstens zwei Bestandteile aufweist, wobei die Anordnung eine zylindrische Kammer (11) aufweist, die Einleitungsmittel zum Einleiten der Bestandteile aufweist, die in der Kammer miteinander zu mischen sind, und Verschlußmittel (23) zum Verschließen der Kammer (11) nach dem Einleiten der genannten Bestandteile, wobei ein Ende der Kammer mittels eines gleitbaren Kolbens (12) zum Ausstoßen den Gemischs abgesperrt wird, und das andere Ende abgesperrt wird durch einen Deckel (15), der einen Anschluß (19) für eine Saugvorrichtung (21) aufweist, wie auch eine zentrale Öffnung (16) für einen abnehmbaren Rührstab (17), der an einer Rührvorrichtung (18) angeschlossen ist, axial beweglich innerhalb der Kammer,
dadurch gekennzeichnet, daß das genannte Einleitungsmittel einen einzigen Einlaß (20) getrennt von der genannten zentralen Öffnung (16) aufweist, mit einem Einführtrichter (22), der wenigstens während eines Einleitungsvorganges hermetisch an den genannten Einlaß (20) angeschlossen ist, und daß die Vakuumvorrichtung dazu dient, einen Luftstrom von der Umgebung durch den Trichter (22), den Einlaß (20), die Kammer (11) und nach vorn zum Anschluß (19) zu erzeugen.

2. Anordnung nach Anspruch 1, dadurch gekennzeichnet, daß die Vakuumvorrichtung ein Reinigungsfilter (26) aufweist, das Aktivkohle sowie ein Sterilfilter umfaßt.

3. Anordnung nach einem der vorausgegangenen Ansprüche, dadurch gekennzeichnet, daß der Anschluß (19) nach dem Demontieren der Drossel (21B) ein Firmament für eine Ejektordüse bildet.

4. Anordnung nach einem der vorausgegangenen Ansprüche, dadurch gekennzeichnet, daß der Einlaß (20) in den Deckel (15) eintaucht.

5. Anordnung nach einem der Ansprüche 1 bis 3, dadurch gekennzeichnet, daß der Einlaß (20) in die zylindrische Wand der Kammer (11) in einem solchen Abstand vom Kolben (12) eintaucht, daß er vom ersten Bestandteil nach dessen Einleiten bedeckt ist.

6. Anordnung nach einem der vorausgegangenen Ansprüche, dadurch gekennzeichnet, daß die Drossel (21B) in der Mischkammer (11) gleitbar ist.

7. Verfahren zum Herstellen eines Knochenzementes, bestehend aus wenigstens zwei Bestandteilen, durch Mischen der Bestandteile, wobei die Bestandteile in eine zylindrische Kammer (11) eingeleitet werden, die nach dem Einleiten der Bestandteile abgesperrt wird, und wobei ein Ende der Kammer abgesperrt wird mittels eines gleitbaren Kolbens (12) zum Ausstoßen des Gemischs und das andere Ende abgesperrt wird durch einen Deckel (15), der einen Anschluß (19) für eine Vakuumvorrichtung (21) aufweist sowie eine zentrale Öffnung (16) für einen abnehmbaren Rührstab (17), der demontierbar an einer Rührvorrichtung (18) befestigt ist, die axial innerhalb der Kammer beweglich ist, dadurch gekennzeichnet, daß das Einleiten der genannten Bestandteile stattfindet mittels eines einzigen Einlasses (20), der von der zentralen Öffnung (16) getrennt ist, mit einem Einführtrichter (22), der wenigstens während eines Einführvorganges hermetisch an den einzigen Einlaß (20) angeschlossen ist, und daß während der Saugung ein Luftstrom erzeugt wird von der Umgebung durch den Trichter (22), den Einlaß (20), die Kammer (11) sowie zum Anschluß (19) hin.

8. Verfahren nach Anspruch 7, dadurch gekennzeichnet, daß das Einleiten der genannten Bestandteile mittels eines Einlasses (20) in der zylindrischen Wand der Kammer (11) stattfindet, und daß sich der Einlaß nach dem Einleiten des ersten Bestandteiles unterhalb des Spiegels dieses ersten Bestandteiles in der Kammer (11) befindet.

## Revendications

1. Dispositif pour la préparation de ciment pour os, lequel ciment pour os comprend au moins deux composants constitutifs, ce dispositif incluant une chambre cylindrique (11), laquelle comprend un moyen d'introduction pour introduire les constituants qui doivent être mélangés dans la chambre et un moyen de fermeture (23) pour fermer la chambre (11) après introduction de ces constituants, dans lequel une extrémité de la chambre est fermée par un piston (12) pouvant coulisser pour l'éjection du mélange, et l'autre extrémité est fermée par un couvercle (15), lequel comprend un raccordement (19) pour un dispositif d'aspiration par le vide (21), ainsi qu'une ouverture centrale (16) pour une tige d'agitateur (17) pouvant être détachée, laquelle est attachée à un dispositif d'agitation (18) susceptible de se déplacer axialement à l'intérieur de la chambre, caractérisé en ce que ce moyen d'introduction comprend une entrée unique (20), séparée de cette ouverture centrale (16), avec un entonnoir d'introduction (22) qui, au moins pendant la phase d'introduction, est raccordé de façon hermétique à cette entrée (20) et que le dispositif d'aspiration par le vide est destiné à produire un courant d'air depuis l'environnement à travers l'entonnoir (22), l'entrée (20) et la chambre (11) et ensuite vers le raccordement (19).

2. Dispositif suivant la revendication 1, caractérisé en ce que le dispositif d'aspiration par le vide comprend un filtre de purification (26) contenant du carbone actif et un filtre stérile.

3. Dispositif suivant l'une quelconque des revendications précédentes, caractérisé en ce que le raccordement (19) après démontage de la buse (21B), forme une base pour un ajutage d'éjection.

4. Dispositif suivant l'une quelconque des revendications précédentes, caractérisé en ce que l'entrée (20) débouche dans le couvercle (15).

5. Dispositif suivant l'une quelconque des revendications 1 à 3, caractérisé en ce que l'entrée (20) débouche dans la paroi cylindrique de la chambre (11) à une distance telle du piston (12) que l'entrée est recouverte par le premier composant constitutif après introduction de ce constituant.

6. Dispositif suivant l'une quelconque des revendications précédentes, caractérisé en ce que la buse (21B) peut coulisser dans la chambre de mélange (11).

7. Procédé pour la préparation de ciment pour os, composé d'au moins deux composants constitutifs, par mélange des constituants, selon lequel les constituants sont introduits dans une chambre cylindrique (11), qui est fermée après introduction de ces constituants, et selon lequel une extrémité de la chambre (11) est fermée par un piston (12) pouvant coulisser pour l'éjection du mélange, et l'autre extrémité est fermée par un couvercle (15), qui comprend un raccordement (19 pour un dispositif d'aspiration par le vide (21), ainsi qu'une ouverture centrale (16) pour une tige d'agitateur (17) pouvant être détachée qui est attachée de façon à pouvoir être démontée à un dispositif d'agitation (18) susceptible de se déplacer axialement à l'intérieur de la chambre, caractérisé en ce que l'introductrion de ces constituants a lieu au moyen d'une entrée unique (20), laquelle entrée unique (20) est séparée de l'ouverture centrale (16), avec un entonnoir d'introduction (22) qui est raccordé de façon hermétique à cette entrée unique (20), au moins pendant la phase d'introduction, et que pendant l'aspiration par le vide, il est produit un courant d'air depuis l'environnement à travers l'entonnoir (22), l'entrée (20) et la chambre (11) et vers le raccordement (19).

8. Procédé suivant la revendication 7, caractérisé en ce que l'introduction de ces constituants a lieu au moyen d'une entrée (20) dans la paroi cylindrique de la chambre (11), après introduction du premier constituant, cette entrée est située en-dessous du niveau du premier constituant dans la chambre (11).
